# EUROPÄISCHE PATENTSCHRIFT

(11) **EP 0 666 068 B1**
(45) Veröffentlichungstag und Bekanntmachung des Hinweises auf die Patenterteilung: **08.09.1999**
(21) Anmeldenummer: 94810065.6
(22) Anmeldetag: 07.02.1994
(51) Int. Cl.: A61F 2/34, A61F 2/30

(54) **Aussenschale zu einer mindestens zweischaligen Gelenkpfanne einer Hüftgelenkprothese und Hüftgelenkprothese mit einer derartigen Aussenschale**
Outer shell a hip joint prosthesis acetabular cup having at least two shells and hip joint prosthesis with such an outer shell
Cupule externe d'une coque acétabulaire d'une prothèse de l'articulation de la hanche à au moins deux cupules et prothèse de l'articulation de la hanche avec une telle cupule externe

(43) Veröffentlichungstag der Anmeldung: 09.08.1995
(73) Patentinhaber: Sulzer Orthopädie AG, 6340 Baar (CH)
(72) Erfinder: Oehy, Jürg, CH-8405 Winterthur (CH); Bider, Kurt, CH-8406 Winterthur (CH); Schoch, Martin, CH-8143 Stallikon (CH)
(74) Vertreter: Trieblnig, Adolf

(56) Entgegenhaltungen:
- EP-A- 0 420 795
- EP-A- 0 444 381
- EP-A- 0 490 616
- EP-A- 0 500 477
- CH-A- 674 927
- FR-A- 2 592 787
- US-A- 4 955 325

## Beschreibung

Die Erfindung betrifft eine Aussenschale zu einer mindestens zweischaligen Gelenkpfanne einer Hüftgelenkprothese, wobei die Aussenschale mehrere Durchtrittsöffnungen aufweist, welche zur Aufnahme von Befestigungselementen zum Befestigen der Aussenschale in einem Knochenbett bestimmt sind und welche je mit einer an der Innenseite der Aussenschale ausgebildeten Ansenkung ausgeführt sind.

Ferner betrifft die Erfindung eine mit einer derartigen Aussenschale ausgeführte Hüftgelenkprothese sowie einen Verschlussstopfen für eine derartige Aussenschale.

Eine aus der CH-PS 674 927 bekannte Gelenkpfanne der genannten Art enthält eine metallische Aussenschale und eine in diese einsetzbare Kunststoff-Innenschale, welche im Bereich der die Aussenschale durchsetzenden Durchtrittsöffnungen mit einer metallischen Trennschicht in Form eines in die Aussenfläche der Innenschale eingesetzten, aus einem Blech geformten Schalenkörpers belegt ist. Durch diese Trennschicht soll ein Kaltfliessen des Kunststoffs der Innenschale in die von Befestigungselementen frei bleibenden Durchtrittsöffnungen der Aussenchale verhindert und damit ein direkter Kontakt zwischen dem Knochengewebe und einem gegebenenfalls im Bereich dieser Durchtrittsöffnungen entstehenden Kunststoffabrieb vermieden werden. Die relativ aufwendige, dreiteilige Konstruktion der bekannten Gelenkpfanne erfordert insbesondere bei Ausführungen, von denen ein Vorrat von mit unterschiedlichen, abgestuften Abmessungen der Aussenschale und der Innenschale bereitgestellt wird, jeweils auch eine Lagerhaltung einer entsprechenden Anzahl unterschiedlicher Schalenkörper für die Ausbildung der entsprechenden Trennschichten.

Der Erfindung liegt die Aufgabe zugrunde, eine insbesondere in dieser Hinsicht verbesserte, vereinfachte Ausführung einer Gelenkpfanne zu schaffen, welche eine von der Ausbildung der Innenschale und von der jeweiligen Grösse der Aussenschale unabhängige Abschirmung des Knochengewebes gegen unerwünschten Kontakt mit Abriebpartikeln gewährleistet.

Diese Aufgabe wird gemäss der Erfindung durch die im Patentanspruchs 1 angegebenen Merkmale gelöst.

Die erfindungsgemässe Bestückung der Aussenschale mit mehreren, je einem der von Befestigungselementen freien Durchtrittsöffnungen zugeordneten Verschlussstopfen gestattet vor dem Einsetzen der Innenschale eine gezielte, je für sich kontrollierbare örtliche Abschirmung jeder einzelnen der im Bereich dieser Durchtrittsöffnungen befindlichen Partien des Knochengewebes gegen den Innenraum der Aussenschale. Die erfindungsgemäss ausgebildeten Verschlussstopfen sind relativ einfach herstellbar und können in einem relativ grossen Vorrat dem Chirurgen bereitgestellt und von diesem auf einfache Weise in die jeweilige Durchtrittsöffnung eingesetzt bzw. aus dieser entfernt werden. Die erfindungsgemässe Ausführung gestattet insbesondere eine von der Form und von der Anbringung der Innenschale unabhängige Abdichtung der Durchtrittsöffnungen in einer vorteilhaft frühen Operationsphase, z.B. vor oder unmittelbar nach dem Einsetzen der Aussenschale in das Knochenbett. Die erfindungsgemäss bestückte Aussenschale ermöglicht ferner die Verwendung einer vorteilhaft einfach ausgeführten Innenschale in einer von der Anordnung der Verschlussstopfen vollständig unabhängigen Formgebung.

Ausgestaltungen des Erfindungsgegenstandes sind in den abhängigen Patentansprüchen angegeben.

Eine mit einer erfindungsgemässen Aussenschale ausgestattete Hüftgelenkprothese ist Gegenstand des Patentanspruchs 7.

Ein Verschlussstopfen für eine erfindungsgemässe Aussenschale ist Gegenstand des Patentanspruchs 8.

Die Erfindung wird anhand eines in der Zeichnung schematisch dargestellten Ausführungsbeispiel erläutert. Es zeigen:
- Fig. 1: Teile einer erfindungsgemäss ausgebildeten Hüftgelenkprothese mit einer Gelenkpfanne in einem diametral verlaufenden Schnitt,
- Fig. 2: eine Einzelheit der Gelenkpfanne nach Fig. 1 in einer grösseren Darstellung, mit einem Verschlussstopfen in einem Schnitt entsprechend der Linie II-II in Fig. 3,
- Fig. 3: den Verschlussstopfen nach Fig. 2 in einer Draufsicht gemäss Pfeil III in Fig. 1.

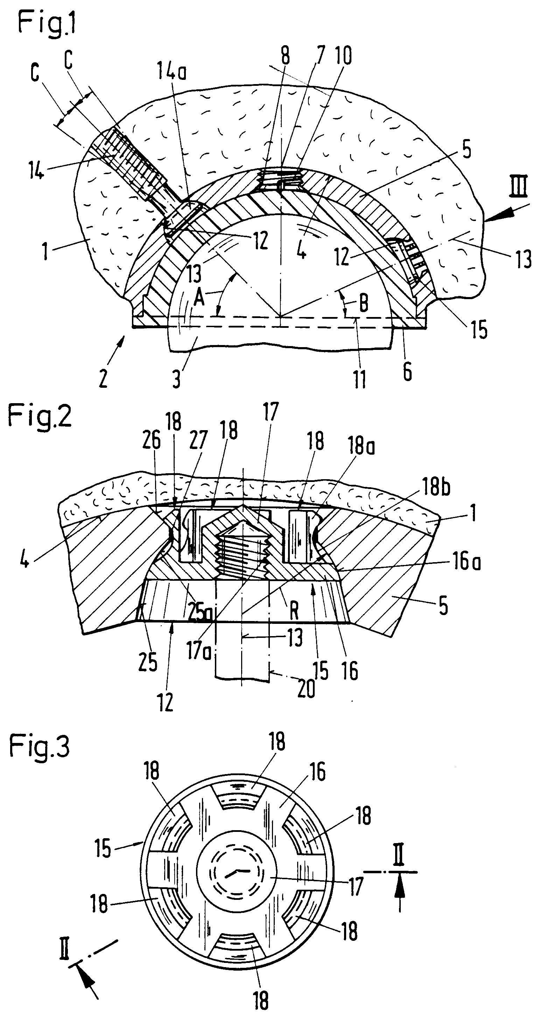

Die Hüftgelenkprothese nach den Fig. 1 und 2 enthält eine in das Knochengewebe 1 eines menschlichen Beckenteils einsetzbare Gelenkpfanne 2 und einen Gelenkkopf 3, der über einen nicht dargestellten Schaftteil in einem Oberschenkelknochen befestigbar ist. Die Gelenkpfanne 2 enthält eine in ein vorbereitetes Knochenbett 4 einführbare und in diesem verankerbare metallische, z.B. aus Titan bestehende Aussenschale 5 im wesentlichen in Form einer hohlen Halbkugel und eine in diese formschlüssig einsetzbare, ebenfalls halbkugelförmige Innenschale 6, welche den Gelenkkopf 3 aufnimmt und welche darstellungsgemäss aus einem Kunststoff, z.B. Polyäthylen, besteht. Nach einer anderen Ausführungsform kann auch eine entsprechende metallische Innenschale vorgesehen sein. Nach einer weiteren, nicht dargestellten Ausführungsform kann zwischen der Aussenschale 5 und der Innenschale 6 auch eine Zwischenschale vorgesehen sein, die aus einem Kunststoff oder einem Metall bestehen kann.

Die Aussenschale ist im Bereich ihres Pols 7 mit einer Gewindebohrung 8 versehen, die zur Aufnahme eines von innen her in sie einschraubbaren Verschlusselements 10 bestimmt ist, welches nicht Gegenstand der vorliegenden Erfindung ist. Zwischem dem Pol 7 und dem Aequator 11 der Aussenschale 5 sind weitere, in Umfangsrichtung gegeneinander versetzte radiale Durchtrittsöffnungen 12 vorgesehen, welche die Aussenschale 5 mit in unterschiedlichen Winkeln A und B zur Ebene des Aequators 11 geneigten Achsen 13 durchsetzen. Die Durchtrittsöffnungen 12 sind je zur Aufnahme eines im Knochengewebe 1 verankerbaren Befestigungselementes 14 beliebiger Art, darstellungsgemäss in Form einer in das Knochengewebe 1 einschraubbaren Knochenschraube, bestimmt. Zur Befestigung der Aussenschale 5 im Knochenbett 4 können auch zwei oder mehr, z.B. drei, entsprechende Befestigungselemente 14 vorgesehen sein. Die nicht benutzten, von Befestigungselementen 14 freien Durchtrittsöffnungen 12 der Aussenschale 6 sind mit von innen her in sie einsetzbaren und feststellbaren Verschlussstopfen 15 versehen, von denen in der Fig. 1 nur einer dargestellt ist.

Entsprechend der Darstellung nach den Fig. 1 und 2 sind die Durchtrittsöffnungen 12 je mit einer inneren Ansenkung 25 und einer äusseren Ansenkung 26 ausgeführt, welche mit der inneren Ansenkung 25 eine örtlich verengte Umfangspartie 27 der Durchtrittsöffnung 12 begrenzt. Wie insbesondere aus der Fig. 2 hervorgeht, können die inneren Ansenkungen 25 der Durchtrittsöffnungen 12 je eine domartige Form aufweisen, die durch einen kegelförmigen inneren Endabschnitt und eine zwischen diesem und der Umfangspartie 27 ausgebildete, zumindest annähernd sphärische Stützfläche 25a bestimmt ist. Die Stützflächen 25a sind gemäss Fig. 2 je mit einem Radius R ausgeführt. Gemäss Fig. 1 können die Befestigungselemente 14 je mit einer Kopfpartie 14a ausgeführt sein, welche eine an die konkave Stützfläche 25a anlegbare, entsprechend konvexe Aufsetzfläche aufweist und welche dementsprechend die Anbringung des Befestigungselementes 14 im Knochengewebe 1 jeweils in einer Winkelstellung zulässt, die derjenigen der Achse 13 der Durchtrittsöffnung 12 entspricht oder die von dieser Achse 13 um einen in Fig. 1 angedeuteten Winkel C abweicht. Die dargestellte Ausführung gestattet somit eine entsprechende, innerhalb dieses Winkelbereichs variable Anpassung an die jeweils gegebenen anatomischen Verhältnisse.

Die Verschlussstopfen 15 enthalten je einen in die innere Ansenkung 25 der jeweils nicht benutzten Durchtrittsöffnungen 12 versenkbare einführbaren tellerartigen Dichtungsteil 16, eine von diesem gegen die Aussenseite der Aussenschale 5 vorstehende, zentrale hutartige Erhebung 17 und eine Anzahl, darstellungsgemäss sechs, von einer Randpartie des Dichtungsteils 16 gegen die Aussenseite der Aussenschale 5 vorstehende, segmentartig angeordnete Biegefedern 18. Der Dichtungsteil 16 ist mit einer über seinen Umfang umlaufenden, an eine Wandpartie der Ansenkung 25, darstellungsgemäss an die Stützfläche 25a, anlegbaren Dichtfläche 16a ausgeführt. Der Verschlussstopfen 15 wird durch die Biegefedern 18 in der Durchtrittsöffnung 12 gehalten und mit der Dichtfläche 16a an die Stützfläche 25a angepresst. Wie insbesondere aus der Fig. 2 hervorgeht, kann die Dichtfläche 16a durch eine der Stützfläche 25a entsprechende sphärische Gegenfläche des Dichtungsteils 16 gebildet sein, welche mit der Stützfläche 25a über eine ringförmige Kontaktfläche zusammenwirkt und damit eine wirksame Abdichtung der Durchtrittsöffnung 12 gewährleistet. Die Erhebung 17 ist mit einer gegen die Innenseite der Aussenschale 5 offenen Sackbohrung 17a ausgeführt, die darstellungsgemäss mit einem Gewinde versehen sein kann und die zur Aufnahme eines Mitnehmerteils eines strichpunktiert angedeuteten Setzwerkzeugs 20 bestimmt ist. Die Biegefedern 18 sind durch aus dem Randbereich des Dichtungsteils 16 nach aussen abstehende, relativ zueinander bewegliche, elastisch verformbare laschenartige Wandsegmente gebildet, welche je einen in die äussere Ansenkung 26 einführbaren, gegen deren Wand verspannbaren Endabschnitt 18a und eine der verengten Umfangspartie 27 der Durchtrittsöffnung 12 entsprechende, diese aufnehmende nutenartige Vertiefung 18b aufweisen.

Die Verschlussstopfen 15, die aus dem gleichen Matrial wie die Aussenschale 5, z.B. aus einer Titanlegierung, bestehen können, können jeweils durch das Setzwerkzeug 20 in die Durchtrittsöffnung 12 eingeführt werden, wobei die Biegefedern 18 beim Uebergang der Endpartien 18a über die Umfangspartie 27 verformt werden und anschliessend mit der Wand der Durchtrittsöffnung 12 eine Schnappverbindung bilden, durch welche der Dichtungsteil 16 mit der Dichtfläche 16a gegen die Stützfläche 18a verspannt und damit die im Bereich der Durchtrittsöffnung 12 befindliche Partie des Knochengewebes 1 gegen die Innenseite der Aussenschale 5 abgedichtet und ein Durchtritt von Abriebpartikeln verhindert wird. Die Verschlussstopfen 15 können über das Setzwerkzeug 20 auf einfache Weise in die Durchtrittsöffnungen 12 eingesetzt und aus diesen entfernt werden. Die beschriebene Ausführung des Verschlusstopfens 15 ermöglicht auch bei relativ dünnen Aussenschalen 5, z.B. solchen mit Wandstärken von 4 bis 5 mm, eine sichere Abdichtung der Durchtrittsöffnungen 12, welche in der Regel für eine Anbringung von Gewinden nicht geeignet sind.

Es sind zahlreiche abgewandelte Ausführungsformen der Erfindung möglich. So kann der Dichtungsteil 16 auch mit einer anders geformten, z.B. zylindrischen oder kegelförmigen, Dichtfläche 16a ausgeführt sein, die zum Zusammenwirken mit der Stützfläche 25a oder mit dem kegeligen Endabschnitt 25 der Ansenkung bestimmt sein kann. Nach einer nicht dargestellten Ausführungsform können die inneren Ansenkungen der Durchtrittsöffnungen 12 je über die ganze Tiefe kegelförmig ausgeführt und zur Aufnahme eines entsprechend kegelförmigen Kopfteils eines Befestigungselementes oder - wie aus der eingangs genannten CH-PS 674 927 bekannt - einer Konushülse bestimmt sein, die ein in unterschiedlichen Winkelstellungen anbringbares Befestigungselement aufnehmen kann. Auch bei dieser Ausführung können die Verschlussstopfen 15 mit an die Ansenkungen 25 flächig anlegbaren kegelförmigen Dichtflächen oder gegebenenfalls mit unter Linienberührung anlegbaren, z.B. zylindrischen Dichtflächen ausgeführt sein. Anstelle der dargestellten Schraubverbindung zwischen der Erhebung 17 und dem Setzwerkzeug 20 kann auch eine andere Mitnehmerverbindung, z.B. eine lösbare Hakenverbindung oder dergleichen, vorgesehen sein. Es versteht sich ferner, dass entsprechende Verschlussstopfen auch je mit einer von der dargestellten Anordnung abweichenden Anzahl z.B. drei, vier, acht oder mehr, Biegefedern 18 in beliebiger Ausführung versehen sein können.

Zusammenfassend lässt sich die Erfindung wie folgt beschreiben:

Die Aussenschale 5 weist mehrere Durchtrittsöffnungen 12 auf, welche zur Aufnahme von Befestigungselementen 14 zum Befestigen der Aussenschale 5 in einem Knochenbett 4 bestimmt und je mit einer inneren Ansenkung 25 ausgeführt sind. Die von Befestigungselementen 14 freien Durchtrittsöffnungen 12 sind je mit einem von innen her einsteckbaren Verschlussstopfen 15 versehen, welche mit einer in der Ansenkung 25 abstützbaren, über diese umlaufenden Dichtfläche 16a ausgeführt ist. Der Verschlusstopfen 15 enthält eine Anzahl in die betreffende Durchtrittsöffnung 12 einführbare Biegefedern 18, die mit der Wand der Durchtrittsöffnung 18 eine Schnappverbindung bilden, und eine im Abstand von den Biegefedern 18 angeordnete, hutartige zentrale Erhebung 17, welche mit einer nach innen offenen Sackbohrung 17a zur Aufnahme eines in diese einschraubbaren Setzwerkzeugs 20 versehen ist. Dadurch wird eine sichere Abdichtung der Durchtrittsöffnungen 12 erzielt und ein Austreten von gegebenenfalls innerhalb der Aussenschale 5 entstehenden Abriebpartikeln in das Knochenbett 4 verhindert.

## Patentansprüche

1. Aussenschale (5) zu einer mindestens zweischaligen Gelenkpfanne (2) einer Hüftgelenkprothese, wobei die Aussenschale (5) mehrere Durchtrittsöffnungen (12) aufweist, welche zur Aufnahme von Befestigungselementen (14) zum Befestigen der Aussenschale (5) in einem Knochenbett (4) bestimmt sind und welche je mit einer an der Innenseite der Aussenschale (5) ausgebildeten Ansenkung (25) ausgeführt sind, dadurch gekennzeichnet, dass nicht benutzte Durchtrittsöffnungen (12) je mit einem von innen her einsteckbaren Verschlussstopfen (15) versehen sind, welcher mit einer in der Ansenkung (25) abstützbaren, über diese umlaufenden Dichtfläche ausgeführt ist und welcher eine Anzahl in die betreffende Durchtrittsöffnung (12) einführbare Biegefedern (18) aufweist, die mit der Wand der Durchtrittsöffnung (12) eine Schnappverbindung bilden.

2. Aussenschale nach Anspruch 1, dadurch gekennzeichnet, dass die Ansenkungen (25) der Durchtrittsöffnung (12) je eine domartige Form aufweisen, die durch eine zumindest annähernd sphärische Stützfläche (25) bestimmt ist, und dass die Dichtflächen (16a) der Verschlussstopfen (15) je an einer an diese sphärische Stützfläche (25a) anlegbaren, entsprechend sphärischen Randpartie des betreffenden Verschlussstopfens (15) ausgebildet sind.

3. Aussenschale nach Anspruch 1 oder 2, dadurch gekennzeichnet, dass die Verschlussstopfen (15) je eine im Abstand von den Biegefedern (18) angeordnete, gegen die Aussenseite der Aussenschale (5) vorstehende, hutartige zentrale Erhebung (17) aufweisen, welche mit einer nach innen offenen Sackbohrung (17a) zur Aufnahme eines in diese einführbaren Mitnehmerteils eines Setzwerkzeugs (20) versehen ist.

4. Aussenschale nach Anspruch 3, dadurch gekennzeichnet, dass die Sackbohrung (17a) mit einem Gewinde versehen ist.

5. Aussenschale nach einem der vorangehenden Ansprüche, dadurch gekennzeichnet, dass die Biegefedern (18) durch aus dem Bereich der Dichtfläche (16a) nach aussen abstehende, relativ zueinander bewegliche laschenartige Wandsegmente des Verschlussstopfens (15) gebildet sind.

6. Aussenschale nach einem der vorangehenden Ansprüche, dadurch gekennzeichnet, dass die Durchtrittsöffnungen (12) je mit einer an der Aussenseite der Aussenschale (5) ausgebildeten äusseren Ansenkung (26) ausgeführt sind, welche mit der an der Innenseite ausgebildeten Ansenkung (25) eine örtlich verengte Umfangspartie (27) der Durchtrittsöffnung (12) begrenzt, und dass die Biegefedern (18) je mit einem in die äussere Ansenkung (26) einführbaren Endabschnitt (18a) und mit einer der verengten Umfangspartie (27) entsprechenden nutenartigen Vertiefung (18b) ausgeführt sind.

7. Hüftgelenkprothese mit einer Aussenschale nach einem der vorangehenden Ansprüche.

## Claims

1. Outer shell (5) for an at least two shelled joint socket (2) of a hip joint prosthesis, wherein the outer shell (5) has a plurality of through-holes (12) which are provided for the receipt of fastener elements (14) for the fastening of the outer shell (5) in a bone seat (4) and which are each executed with a counter-sink (25) formed on the inner side of the outer shell (5), characterized in that the through-holes (12) which are not utilised are each provided with a closure plug (15) which is insertable from the inside and designed with a sealing surface supportable in and extending around the counter-sink (25), and which further comprises a number of flexible springs (18) which are insertable into the relevant through-hole (12) and form a snap connection with the wall of the through-hole (12).

2. Outer shell in accordance with claim 1, characterized in that the counter-sinks (25) of the through-hole (12) each have a dome-like shape determined by an at least approximately spherical support surface (25), and in that the sealing surfaces (16a) of the closure plugs (15) are each formed on a correspondingly spherical edge portion of the relevant closure plug (15) which can be seated on this spherical supporting surface (25a).

3. Outer shell in accordance with claim 1 or 2, characterized in that the closure plugs (15) each comprise a hat-like central projection (17) disposed spaced from the flexible springs (18) and protruding towards the outer side of the outer shell (5), the central projection being provided with an inwardly open blind bore (17a) for the receipt of a driver part of a placement tool (20) introduceable into the blind bore (17a).

4. Outer shell in accordance with claim 3, characterized in that the blind bore (17a) is provided with a thread.

5. Outer shell in accordance with one of the preceding claims, characterized in that the flexible springs (18) are formed by lug-like wall segments of the closure plug (15) which are moveable relative to one another and which protrude outwardly from the region of the sealing surface (16a).

6. Outer shell in accordance with one of the preceding claims, characterized in that the through-holes (12) are each designed with an outer counter-sink (26) formed on the outer side of the outer shell (5) which, together with the counter-sink (25) formed on the inner side, bounds a locally narrowed circumferential part (27) of the through-hole (12), and in that the flexible springs (18) each formed with an end portion (18a) insertable into the outer counter-sink (26) and a groove-like recess (18b) corresponding to the narrowed circumferential part (27).

7. Hip joint prosthesis comprising an outer shell in accordance with one of the preceding claims.

## Revendications

1. Cupule extérieure (5) d'une coque acétabulaire (2) à au moins deux cupules d'une prothèse de l'articulation de la hanche, où la cupule extérieure (5) présente plusieurs ouvertures traversantes (12) qui sont destinées à recevoir des éléments de fixation (14) pour fixer la cupule externe (5) dans un lit osseux (4) et qui sont réalisées chacune avec un chanfrein (25) réalisé au côté intérieur de la cupule extérieure (5), caractérisée en ce que des ouvertures traversantes (12) non utilisées sont pourvues chacune d'un bouchon de fermeture (15) pouvant être inséré de l'intérieur qui est réalisé avec une face d'étanchéité prenant appui sur le chanfrein (25), s'étendant autour de celui-ci et qui présente un nombre de ressorts spiraux (18) pouvant être insérés dans l'ouverture traversante concernée (12), qui forment avec la paroi de l'ouverture traversante (12) une liaison à enclenchement.

2. Cupule externe selon la revendication 1, caractérisée en ce que les chanfreins (25) de l'ouverture traversante (12) présentent chacun une forme en dôme par laquelle est définie une face d'appui (25) au moins approximativement sphérique, et en ce que les faces d'étanchéité (16a) des bouchons de fermeture (15) sont réalisées chacune à une partie de bord sphérique correspondante, applicable à cette face d'appui sphérique (25a) du bouchon de fermeture concerné (15).

3. Cupule externe selon la revendication 1 ou 2, caractérisée en ce que les bouchons de fermeture (15) présentent chacun une surélévation centrale en forme de chapeau (7), disposée à une certaine distance des ressorts spiraux (18), dépassant vers le côté extérieur de la cupule externe (5), qui est pourvue d'un trou borgne (17a) ouvert vers l'intérieur pour recevoir une partie d'entraînement insérable dans celui-ci d'un outil de pose (20).

4. Cupule externe selon la revendication 3, caractérisée en ce que le trou borgne (17a) est pourvu d'un filetage.

5. Cupule externe selon l'une des revendications précédentes, caractérisée en ce que les ressorts spiraux (18) sont formés par des segments de paroi en forme de languette du bouchon de fermeture (15), mobiles les uns relativement aux autres, dépassant de la zone de la face d'étanchéité (16a) vers l'extérieur.

6. Cupule externe selon l'une des revendications précédentes, caractérisée en ce que les ouvertures traversantes (12) sont réalisées chacune avec un chanfrein, extérieur (26) réalisé au côté extérieur de la cupule externe (5), qui délimite avec le chanfrein (25) réalisé au côté interne une partie périphérique (27) localement rétrécie de l'ouverture de passage (12), et en ce que les ressorts spiraux (18) sont réalisés chacun avec un tronçon d'extrémité (18a) insérable dans le chanfrein extérieur (26) et avec un creux en forme de rainure (18b) correspondant à la partie périphérique rétrécie (27).

7. Prothèse de l'articulation de la hanche avec une cupule extérieure selon l'une des revendications précédentes.
